# EUROPEAN PATENT APPLICATION

(11) **EP 4 538 381 A1**
(43) Date of publication of application: **16.04.2025**
(21) Application number: 23839985.1
(22) Date of filing: 13.07.2023
(51) Int. Cl.: C12N 15/88, C07K 14/005, C07K 14/47, A61K 39/12, A61K 39/00, A61P 31/12, A61P 35/00, A23L 33/13

(54) **EXTRACELLULAR VESICLE COMPRISING ANTIGENIC PROTEIN OR GENE ENCODING SAME PROTEIN, AND USES THEREOF**

(30) Priority: 13.07.2022 KR 20220086472
(71) Applicant: Ewha University-Industry Collaboration Foundation, Seoul 03760 (KR); Exollence Co., Ltd., Seoul 07985 (KR)
(72) Inventor: KWON, Ki Hwan, Seoul 06009 (KR); CHUNG, Ji Hwa, Gimpo-si Gyeonggi-do 10083 (KR); KIM, Kyoung Hwa, Seoul 07572 (KR)
(74) Representative: Vossius & Partner Patentanwälte Rechtsanwälte mbB
(86) International application number: PCT/KR2023/010004
(87) International publication number: WO 2024/014894

(57) **Abstract**

The present disclosure relates to an extracellular vesicle including an antigen protein or a gene encoding the antigen protein and use thereof, and more particularly, to: an extracellular vesicle including an antigen protein derived from a virus, a microorganism, or cancer cells, or a gene encoding the antigen protein; or a vaccine composition for the prevention or treatment of a viral infection, a microbial infection, or cancer, the vaccine composition including the extracellular vesicle. The extracellular vesicle or the vaccine composition including the extracellular vesicle, according to the present disclosure, is a platform that has stability and an excellent effect of inducing an antigen-specific immune response and can be applied to various diseases, and thus is expected to be effectively used in the field of development of a vaccine for the prevention or treatment of various diseases, including microbial infections or cancer.

## Description

### Technical Field

The present disclosure relates to an extracellular vesicle including an antigen protein or a gene encoding the antigen protein and use thereof.

### Background Art

A vaccine is a drug that imparts acquired immunity against a certain disease or pathogen in animals, including humans. A vaccine has a structure similar to the antigen recognition site of a microbial pathogen that mainly cause diseases, but has no pathogenicity unlike pathogens. Recently, various types of vaccines for various pathogens and various diseases have been developed, but there are still many diseases that require the development of more effective and safe vaccines.

Extracellular vesicles are nanovesicles consisting of lipid bilayers and having a size of several tens to hundreds of nanometers, and consist of biologically active substances such as proteins, lipids, and genes. Representative examples of these extracellular vesicles include exosomes, ectosomes, microvesicles, bacterial outer membrane vesicles (bacterial OMVs), microorganism-derived extracellular vesicles including yeast-derived extracellular vesicles, apoptotic bodies, or the like. Previously, these extracellular vesicles were considered debris secreted from cells, but recently, as the clinical significance of extracellular vesicles has emerged, various studies have been conducted. In particular, exosomes, which are spherical vesicles released by cells, contain various types of information such as proteins and DNAs of parent cells, and thus the development of cancer diagnostic markers and sensors using the same as biomarkers is actively ongoing.

Meanwhile, exosomes are small membrane-structured vesicles (approximately 30 nm to 100 nm in diameter) secreted from various cells. In studies through electron microscopy, it was observed that exosomes did not separate directly from the plasma membrane, but originated from specific compartments inside cells called multivesicular bodies (MVBs), and were released and secreted out of the cells. In other words, when the MVBs are fused with the plasma membrane, small vesicles are released into the extracellular environment, which are referred to as exosomes. These exosomes are known to be produced and secreted in a living state from not only red blood cells but also various types of immune cells, including B-lymphocytes, T-lymphocytes, dendritic cells, platelets, macrophages, and the like, tumor cells, stem cells, and the like.

Mammalian cell-derived exosomes are known to be involved in various physiological functions such as hemostasis, tissue regeneration, stem cell maintenance, inflammatory/immune response regulation, and embryonic development. In particular, it has been reported that exosomes derived from immune cells may deliver inflammatory cytokines, or may directly or indirectly present an antigen to promote an immune response ((Nat Rev Immunol. 2009 Aug;9(8):581-93). Based on the characteristics and *in vivo* functions of these exosomes, the exosomes are being researched and developed for use as nano-sized substance delivery systems, mediators and biomarkers in the fields of oncology, immunotherapy, regenerative medicine, and the like.

As such, studies are being conducted for use of exosomes or extracellular vesicles, including microorganism-derived extracellular vesicles, as a vaccine, but are still restricted to some diseases. In addition, research into and development of vaccine platform technology applicable to various diseases, including viral or microbial infectious diseases or cancer, are insufficient.

### Disclosure

### Technical Problem

As a result of having researched and made efforts to develop an extracellular vesicle-based vaccine platform that can be widely applied to various diseases, the inventors of the present disclosure confirmed that an extracellular vesicle serves as a powerful immunostimulant and an antigen carrier to induce the production of an antigen-specific antibody and a specific T cell response, thereby completing the present disclosure.

Therefore, the objective of the present disclosure is to provide an extracellular vesicle including an antigen protein or a gene encoding the antigen protein.

Also, another objective of the present disclosure is to provide a vaccine composition for preventing or treating a viral infection, including an extracellular vesicle which includes a virus-derived antigen protein or a gene encoding the virus-derived antigen protein.

Also, another objective of the present disclosure is to provide a vaccine composition for preventing or treating a microbial infection, including an extracellular vesicle which includes a microorganism-derived antigen protein or a gene encoding the microorganism-derived antigen protein.

Also, another objective of the present disclosure is to provide a vaccine composition for preventing or treating cancer, including an extracellular vesicle which includes a cancer cell-derived antigen protein or a gene encoding the cancer cell-derived antigen protein.

Also, another objective of the present disclosure is to provide a health functional food for preventing or ameliorating a viral infection, including an extracellular vesicle which includes a virus-derived antigen protein or a gene encoding the virus-derived antigen protein.

Also, another objective of the present disclosure is to provide a health functional food for preventing or ameliorating a microbial infection, including an extracellular vesicle which includes a microorganism-derived antigen protein or a gene encoding the microorganism-derived antigen protein.

Also, another objective of the present disclosure is to provide a health functional food for preventing or ameliorating cancer, including an extracellular vesicle which includes a cancer cell-derived antigen protein or a gene encoding the cancer cell-derived antigen protein.

Also, another objective of the present disclosure is to provide a method of preventing or treating a viral infection, including a step of administering an extracellular vesicle including a virus-derived antigen protein or a gene encoding the virus-derived antigen protein to a subject in need thereof.

Also, another objective of the present disclosure is to provide a method of preventing or treating a microbial infection, including a step of administering an extracellular vesicle including a microorganism-derived antigen protein or a gene encoding the microorganism-derived antigen protein to a subject in need thereof.

Also, another objective of the present disclosure is to provide a method of preventing or treating cancer, including a step of administering an extracellular vesicle including a cancer cell-derived antigen protein or a gene encoding the cancer cell-derived antigen protein to a subject in need thereof.

Also, another objective of the present disclosure is to provide use of an extracellular vesicle for the preparation of a drug for preventing or treating a viral infection, the extracellular vesicle including a virus-derived antigen protein or a gene encoding the virus-derived antigen protein.

Also, another objective of the present disclosure is to provide use of an extracellular vesicle for the preparation of a drug for preventing or treating a microbial infection, the extracellular vesicle including a microorganism-derived antigen protein or a gene encoding the microorganism-derived antigen protein.

Also, another objective of the present disclosure is to provide use of an extracellular vesicle for the preparation of a drug for the prevention or treatment of cancer, the extracellular vesicle including a cancer cell-derived antigen protein or a gene encoding the cancer cell-derived antigen protein.

However, the technical problems to be achieved by the present disclosure are not limited to the problems mentioned above, and unmentioned other problems will be clearly understood from the following description by those of ordinary skill in the art.

### Technical Solution

To achieve the above-described objectives of the present disclosure, the present disclosure provides an extracellular vesicle including an antigen protein or a gene encoding the antigen protein.

In an embodiment of the present disclosure, the antigen protein may be derived from one or more selected from the group consisting of a virus, a microorganism, and cancer cells.

In another embodiment of the present disclosure, the virus may be one or more selected from the group consisting of adenovirus, smallpox virus, poliovirus, measles virus, hepatitis C virus, human immunodeficiency virus-1 (HIV-1), hepatitis B virus (HBV), influenza virus, respiratory syncytial virus, herpes simplex virus, human papilloma virus, zika virus, varicella-zoster virus, and severe fever with thrombocytopenia syndrome virus.

In another embodiment of the present disclosure, the antigen protein may be one or more selected from the group consisting of a human immunodeficiency virus-1-derived p24 protein, a hepatitis B virus-derived s protein, an influenza virus-derived H1N1 protein, a respiratory syncytial virus-derived RSV-F protein, and varicella-zoster virus-derived glycoprotein E (VZE).

In another embodiment of the present disclosure, the microorganism may be a microorganism of one or more genera selected from *Salmonella, Yersinia, Escherichia, Chlamydia, Xanthomonas, Erwinia, Pseudomonas, Ralstonia, Vibrio, Neisseria, Mycobacterium, Streptococcus, Staphylococcus, Enterococcus, Lactobacillus, Aspergillus, Blastomyces, Ajellomyces, Candida, Coccidioides, Cryptococcus, Histoplasma, Rhizopus, Mucor, Cunninghamella, Apophysomyces, Absidia, Saksenaea, Entomophthora, Conidiobolus, Basidiobolus, Sporothrix, Pneumocystis, Talaromyces, Asclepias, Fusarium, Scedosporium,* and *Mucorales.*

In another embodiment of the present disclosure, the cancer cells may be derived from one or more cancers selected from the group consisting of head and neck cancer, melanoma, leukemia, breast cancer, ovarian cancer, bladder cancer, prostate cancer, lung cancer, colon cancer, and glioblastoma.

In another embodiment of the present disclosure, the antigen protein may be one or more selected from the group consisting of MAGE 1/2/3, WT1, CDK4, MUC-1, HER2, PSA, HPV16 E6/E7, and HCV.

In another embodiment of the present disclosure, the antigen protein or the gene encoding the antigen protein may be loaded into the extracellular vesicle.

In another embodiment of the present disclosure, the extracellular vesicle may be derived from one or more selected from the group consisting of animal cells, plant cells, or a microorganism.

In another embodiment of the present disclosure, the animal cells may be one or more selected from the group consisting of somatic cells, germ cells, immune cells, neurons, and tumor cells.

In another embodiment of the present disclosure, the extracellular vesicle may be derived from activated immune cells or a bacterial outer membrane.

In another embodiment of the present disclosure, when the extracellular vesicle is derived from animal cells, the extracellular vesicle may express one or more proteins selected from the group consisting of CD9, CD63, CD81, Alix, TSG101, Syntenin 1, and Flotillin 1.

In another embodiment of the present disclosure, when the extracellular vesicle is derived from plant cells, the extracellular vesicle may express one or more proteins selected from the group consisting of Syntaxin (PEN1), Tetraspanin 8 (Tet8), and Heat shock protein (HSP).

In another embodiment of the present disclosure, when the extracellular vesicle is derived from a microorganism, the extracellular vesicle may express one or more proteins selected from the group consisting of OmpA, Flagellin, HSP70, and beta-glucan.

Also, an embodiment of the present disclosure provides a vaccine composition for preventing or treating a viral infection, including an extracellular vesicle which includes a virus-derived antigen protein or a gene encoding the virus-derived antigen protein.

In another embodiment of the present disclosure, the viral infection may be one or more selected from the group consisting of adenovirus infection, smallpox virus infection, poliovirus infection, measles virus infection, hepatitis C virus infection, human immunodeficiency virus-1 (HIV-1) infection, hepatitis B virus (HBV) infection, influenza virus infection, respiratory syncytial virus infection, herpes simplex virus infection, human papilloma virus infection, zika virus infection, varicella-zoster virus infection, and severe fever with thrombocytopenia syndrome virus infection.

An embodiment of the present disclosure provides a vaccine composition for preventing or treating a microbial infection, including an extracellular vesicle which includes a microorganism-derived antigen protein or a gene encoding the microorganism-derived antigen protein.

In another embodiment of the present disclosure, the microbial infection may be an infection caused by a microorganism of one or more genera selected from *Salmonella, Yersinia, Escherichia, Chlamydia, Xanthomonas, Erwinia, Pseudomonas, Ralstonia, Vibrio, Neisseria, Mycobacterium, Streptococcus, Staphylococcus, Enterococcus, Lactobacillus, Aspergillus, Blastomyces, Ajellomyces, Candida, Coccidioides, Cryptococcus, Histoplasma, Rhizopus, Mucor, Cunninghamella, Apophysomyces, Absidia, Saksenaea, Entomophthora, Conidiobolus, Basidiobolus, Sporothrix, Pneumocystis, Talaromyces, Asclepias, Fusarium, Scedosporium,* and *Mucorales.*

Also, an embodiment of the present disclosure provides a vaccine composition for preventing or treating cancer, including an extracellular vesicle which includes a cancer cell-derived antigen protein or a gene encoding the cancer cell-derived antigen protein.

In another embodiment of the present disclosure, the cancer may be one or more selected from the group consisting of head and neck cancer, melanoma, leukemia, breast cancer, ovarian cancer, bladder cancer, prostate cancer, lung cancer, colon cancer, and glioblastoma.

In another embodiment of the present disclosure, the vaccine composition may simultaneously induce the production of an antigen-specific antibody and a T cell-mediated immune response.

In another embodiment of the present disclosure, the vaccine composition may be freeze-dried.

Also, an embodiment of the present disclosure provides a health functional food for preventing or ameliorating a viral infection, including an extracellular vesicle which includes a virus-derived antigen protein or a gene encoding the virus-derived antigen protein.

Also, an embodiment of the present disclosure provides a health functional food for preventing or ameliorating a microbial infection, including an extracellular vesicle which includes a microorganism-derived antigen protein or a gene encoding the microorganism-derived antigen protein.

Also, an embodiment of the present disclosure provides a health functional food for preventing or ameliorating cancer, including an extracellular vesicle which includes a cancer cell-derived antigen protein or a gene encoding the cancer cell-derived antigen protein.

Also, an embodiment of the present disclosure provides a method of preventing or treating a viral infection, including a step of administering an extracellular vesicle including a virus-derived antigen protein or a gene encoding the virus-derived antigen protein to a subject in need thereof.

Also, an embodiment of the present disclosure provides a method of preventing or treating a microbial infection, including a step of administering an extracellular vesicle including a microorganism-derived antigen protein or a gene encoding the microorganism-derived antigen protein to a subject in need thereof.

Also, an embodiment of the present disclosure provides a method of preventing or treating cancer, including a step of administering an extracellular vesicle including a cancer cell-derived antigen protein or a gene encoding the cancer cell-derived antigen protein to a subject in need thereof.

Also, an embodiment of the present disclosure provides use of an extracellular vesicle for the preparation of a drug for preventing or treating a viral infection, the extracellular vesicle including a virus-derived antigen protein or a gene encoding the virus-derived antigen protein.

Also, an embodiment of the present disclosure provides use of an extracellular vesicle for the preparation of a drug for preventing or treating a microbial infection, the extracellular vesicle including a microorganism-derived antigen protein or a gene encoding the microorganism-derived antigen protein.

Also, an embodiment of the present disclosure provides use of an extracellular vesicle for the preparation of a drug for preventing or treating cancer, the extracellular vesicle including a cancer cell-derived antigen protein or a gene encoding the cancer cell-derived antigen protein.

### Advantageous Effects

In the present disclosure, it was experimentally confirmed that, when mice were immunized via administration of activated immune cell- or bacterial outer membrane-derived extracellular vesicles loaded with an antigen protein or mRNA, the extracellular vesicles exhibited the effect of inducing the production of an antigen-specific antibody and a T cell response. Thus, an extracellular vesicle or a vaccine composition including the extracellular vesicle, according to the present disclosure, is a platform that has stability and an excellent effect of inducing an antigen-specific immune response and can be applied to various diseases, and thus is expected to be effectively used in the field of development of a vaccine for the prevention or treatment of various diseases, including microbial infections or cancer.

### Description of Drawings

FIG. 1 illustrates the results of confirming that THP-1 cells activated by treatment with LPS exhibited the phenotype of M1 macrophages, in which FIG. 1A is a microscope image showing the morphology of non-activated THP-1 cells (Resting THP-1) and activated THP-1 cells (Activated THP-1), and FIG. 1B illustrates the results of measuring the mRNA levels of IL-1b, IL-6, IL-8, TNF-α, and iNOS, which are pro-inflammatory cytokines, in each case of THP-1 cells.
FIG. 2 illustrates the results of analyzing the characteristics of exosomes isolated from activated THP-1 cells, in which FIG. 2A illustrates the results of measuring the diameters of the isolated exosomes through nanosight tracking analysis (NTA), FIG. 2B is a cryo-electron microscopy (cryo-EM) image showing the exosomes, and FIG. 2C illustrates the results of measuring the expression levels of CD63, CD81, Syntenin 1, and Flotillin 1 proteins, which are exosome markers, by western blotting using a cell lysate and the isolated exosomes.
FIG. 3 illustrates the results of analyzing the characteristics of outer membrane vesicles isolated from attenuated bacteria, wherein the diameters of the isolated bacterial outer membrane vesicles were measured through nanosight tracking analysis (NTA).

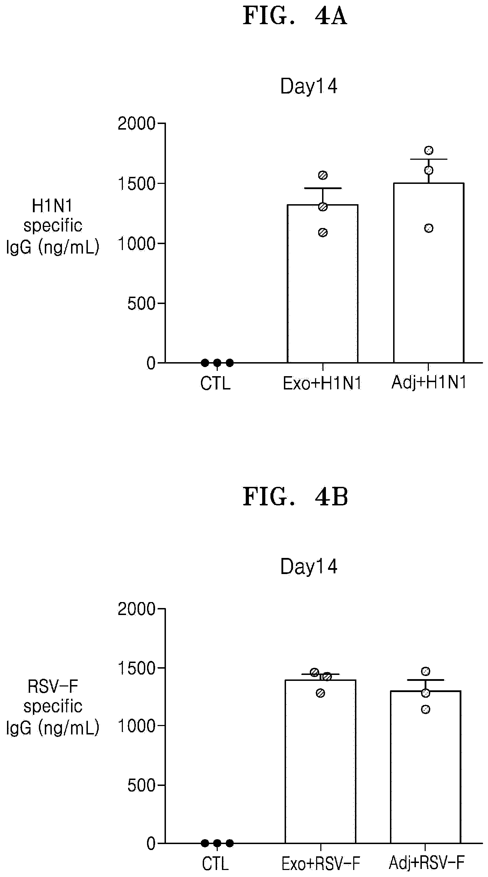

FIG. 4 illustrates the degree of antibody binding in a serum specific to the H1N1 protein or RSV-F protein of immune cell (THP-1 cell)-derived exosomes loaded with the H1N1 protein or the RSV-F protein, in which (1) FIG. 4A illustrates the results of analyzing, through absorbance measurement, the degree of antibody binding in a serum specific to an influenza virus-derived H1N1 protein, after THP-1 cell-derived exosomes or an adjuvant and the H1N1 protein were administered together and the serum was separated after 14 days, and (2) FIG. 4B illustrates the results of analyzing, through absorbance measurement, the degree of antibody binding in a serum specific to a respiratory syncytial virus-derived RSV-F protein, after THP-1 cell-derived exosomes or an adjuvant and the RSV-F protein were administered together and the serum was separated after 14 days.
FIG. 5 depicts an experimental schedule for the analysis of a serum antibody response and specific T cell response to extracellular vesicles loaded with mRNA of a virus-derived protein.
FIG. 6 is a graph showing the results of serum antibody response analysis of mice administered with extracellular vesicles loaded with influenza virus-derived H1N1 mRNA.
FIG. 7 is a graph showing the results of serum antibody response analysis of mice administered with extracellular vesicles loaded with varicella-zoster virus-derived glycoprotein E mRNA.
FIG. 8 is a graph showing the results of serum antibody response analysis of mice administered with extracellular vesicles loaded with respiratory syncytial virus-derived RSV-F mRNA.
FIG. 9 is a graph showing the results of specific T cell response analysis of mice administered with extracellular vesicles loaded with influenza virus-derived H1N1 mRNA.
FIG. 10 is a graph showing the results of specific T cell response analysis of mice administered with extracellular vesicles loaded with varicella-zoster virus-derived glycoprotein E mRNA.

### Best Mode

The present disclosure relates to a vaccine platform technology that can be effectively used in the field of development of a vaccine for the prevention or treatment of various infectious diseases or cancer by using an extracellular vesicle which functions as a powerful immunostimulant and an antigen carrier.

Hereinafter, the present disclosure will be described in detail.

The present disclosure provides an extracellular vesicle including an antigen protein or a gene encoding the antigen protein.

The term "antigen" as used herein refers to a molecule capable of inducing an immune response to produce an antibody in a host subject. Antigens are targets of antibodies, and each antibody is produced in an antigen-specific manner to respond to an antigen after cells of the immune system come into contact with the antigen. In the present disclosure, when the antigen is in a form capable of inducing an immune response as described above, the antigen may include all of proteins, genes encoding the proteins, and mRNA forms of the genes, and may include fragments of the proteins or mRNA forms. The origin of the antigen is not particularly limited, and non-limiting examples of the antigen may include all types of virus-derived antigens, antigens derived from microorganisms including bacteria, archaebacteria, and fungi, and cancer cell-derived antigens.

That is, in the present disclosure, the antigen protein may be derived from one or more selected from the group consisting of a virus, a microorganism, and cancer cells.

The virus may be one or more selected from the group consisting of adenovirus, smallpox virus, poliovirus, measles virus, hepatitis C virus, human immunodeficiency virus-1 (HIV-1), hepatitis B virus (HBV), influenza virus, respiratory syncytial virus, herpes simplex virus, human papilloma virus, zika virus, varicella-zoster virus, and severe fever with thrombocytopenia syndrome virus. Specifically, the virus may be one or more selected from the group consisting of influenza virus, respiratory syncytial virus, herpes simplex virus, human papilloma virus, zika virus, varicella-zoster virus, and severe fever with thrombocytopenia syndrome virus. More specifically, the virus may be one or more selected from the group consisting of influenza virus, varicella-zoster virus, and respiratory syncytial virus.

In the present disclosure, when the antigen protein is derived from a human immunodeficiency virus, the antigen protein may be a p24 protein derived from human immunodeficiency virus-1.

Also, in the present disclosure, when the antigen protein is derived from hepatitis B virus, the antigen protein may be an s protein derived from hepatitis B virus.

Also, in the present disclosure, when the antigen protein is derived from influenza virus, the antigen protein may be one or more selected from the group consisting of a H1N1 protein, a H2N2 protein, a H3N2 protein, a H5N1 protein, a H7N7 protein, a H7N9 protein, a H9N2 protein, and a H10N7 protein, which are derived from influenza virus. Specifically, the antigen protein may be an influenza virus-derived H1N1 protein. More specifically, the antigen protein may be a polypeptide consisting of the amino acid sequence of SEQ ID NO: 1.

The polypeptide consisting of the amino acid sequence of SEQ ID NO: 1 may include a polypeptide having a sequence homology of about 70% or more, about 75% or more, about 80% or more, about 85% or more, about 90% or more, about 92% or more, about 95% or more, about 97% or more, about 98% or more, or about 99% or more to the amino acid sequence of SEQ ID NO: 1, and the polypeptide consisting of the amino acid sequence of SEQ ID NO: 1 may include the amino acid sequence of SEQ ID NO: 1 or a functional variant thereof. A functional variant refers to any similar sequence in which some amino acid substitutions occur at amino acid positions without affecting the biological properties of the polypeptide consisting of the amino acid sequence of SEQ ID NO: 1.

Also, in the present disclosure, when the antigen protein is derived from respiratory syncytial virus, the antigen protein may be an RSV-F protein derived from respiratory syncytial virus, and specifically, may be a polypeptide consisting of the amino acid sequence of SEQ ID NO: 2.

In addition, the polypeptide consisting of the amino acid sequence of SEQ ID NO: 2 may include a polypeptide having a sequence homology of about 70% or more, about 75% or more, about 80% or more, about 85% or more, about 90% or more, about 92% or more, about 95% or more, about 97% or more, about 98% or more, or about 99% or more to the amino acid sequence of SEQ ID NO: 2, and the polypeptide consisting of the amino acid sequence of SEQ ID NO: 2 may include the amino acid sequence of SEQ ID NO: 2 or a functional variant thereof.

Also, in the present disclosure, when the antigen protein is derived from varicella-zoster virus, the antigen protein may be glycoprotein E derived from varicella-zoster virus, and specifically, may be a polypeptide consisting of the amino acid sequence of SEQ ID NO: 3.

In addition, the polypeptide consisting of the amino acid sequence of SEQ ID NO: 3 may include a polypeptide having a sequence homology of about 70% or more, about 75% or more, about 80% or more, about 85% or more, about 90% or more, about 92% or more, about 95% or more, about 97% or more, about 98% or more, or about 99% or more to the amino acid sequence of SEQ ID NO: 3, and the polypeptide consisting of the amino acid sequence of SEQ ID NO: 3 may include the amino acid sequence of SEQ ID NO: 3 or a functional variant thereof.

The term "homology" is intended to indicate sequence similarity to the wild-type amino acid sequence. Comparison in the homology may be performed using a comparison program widely known in the art, and the homology between two or more sequences may be calculated as a percentage (%).

In the present disclosure, the microorganism includes both a prokaryotic microorganism and a eukaryotic microorganism, the prokaryotic microorganism includes eubacteria (bacteria) and archaea, and the eukaryotic microorganism includes fungi, algae, and protozoa. In addition, the eubacteria (bacteria) include both gram-positive bacteria and gram-negative bacteria, and the fungi include both mold and yeast. Specifically, the microorganism may be a microorganism of one or more genera selected from the group consisting of *Salmonella, Yersinia, Escherichia, Chlamydia, Xanthomonas, Erwinia, Pseudomonas, Ralstonia, Vibrio, Neisseria, Mycobacterium, Streptococcus, Staphylococcus, Enterococcus, Lactobacillus, Aspergillus, Blastomyces, Ajellomyces, Candida, Coccidioides, Cryptococcus, Histoplasma, Rhizopus, Mucor, Cunninghamella, Apophysomyces, Absidia, Saksenaea, Entomophthora, Conidiobolus, Basidiobolus, Sporothrix, Pneumocystis, Talaromyces, Asclepias, Fusarium, Scedosporium,* and *Mucorales.*

In addition, in the present disclosure, the cancer cells may be derived from one or more cancers selected from the group consisting of: acoustic neuroma; adenocarcinoma; adrenal gland cancer; anal cancer; angiosarcoma (e.g., lymphangiosarcoma, lymphangioendotheliosarcoma, hemangiosarcoma); appendix cancer; benign monoclonal gammopathy; biliary cancer (e.g., cholangiocarcinoma); bladder cancer; breast cancer (e.g., adenocarcinoma of the breast, papillary carcinoma of the breast, mammary cancer, medullary carcinoma of the breast); brain cancer (e.g., meningioma, glioblastoma, glioblastoma (e.g., astrocytoma, oligodendroglioma), medulloblastoma); bronchus cancer; carcinoid tumor; cervical cancer (e.g., cervical adenocarcinoma); choriocarcinoma; chordoma; craniopharyngioma; colorectal cancer (e.g., colon cancer, rectal cancer, colorectal adenocarcinoma); connective tissue cancer; epithelial carcinoma; ependymoma; endotheliosarcoma (e.g., Kaposi's sarcoma, multiple idiopathic hemorrhagic sarcoma); endometrial cancer (e.g., uterine cancer, uterine sarcoma); esophageal cancer (e.g., adenocarcinoma of the esophagus, Barrett's adenocarcinoma); Ewing's sarcoma; ocular cancer (e.g., intraocular melanoma, retinoblastoma); familiar hypereosinophilia; gall bladder cancer; gastric cancer (e.g., stomach adenocarcinoma); gastrointestinal stromal tumor (GIST); germ cell cancer; head and neck cancer (e.g., head and neck squamous cell carcinoma), oral cancer (e.g., oral squamous cell carcinoma); throat cancer (e.g., laryngeal cancer, pharyngeal cancer, nasopharyngeal cancer, oropharyngeal cancer); heavy chain disease (e.g., alpha chain disease, gamma chain disease, mu chain disease); hemangioblastoma; hypopharynx cancer; inflammatory myofibroblastic tumors; immunocytic amyloidosis; kidney cancer (e.g., nephroblastoma, also known as Wilms' tumor, renal cell carcinoma); liver cancer (e.g., hepatocellular cancer (HCC), malignant hepatoma); lung cancer (e.g., bronchogenic carcinoma, small cell lung cancer (SCLC), non-small cell lung cancer (NSCLC), adenocarcinoma of the lung); leiomyosarcoma (LMS); mastocytosis (e.g., systemic mastocytosis); muscle cancer; myelodysplastic syndrome (MDS); mesothelioma; myeloproliferative disorder (MPD) (e.g., polycythemia vera (PV), essential thrombocytosis (ET), agnogenic myeloid metaplasia (AMM) of unknown etiology, also known as myelofibrosis (MF), chronic idiopathic myelofibrosis, chronic myelocytic leukemia (CML), chronic neutrophilic leukemia (CNL), hypereosinophilic syndrome (HES); neuroblastoma; neurofibroma (e.g., neurofibromatosis (NF) type 1 or type 2, schwannomatosis; neuroendocrine cancer (e.g., gastroenteropancreatic neuroendoctrine tumor (GEP-NET), carcinoid tumor; osteosarcoma (e.g., bone cancer); ovarian cancer (e.g., cystadenocarcinoma, ovarian embryonal carcinoma, ovarian adenocarcinoma); papillary adenocarcinoma; pancreatic cancer (e.g., pancreatic andenocarcinoma, intraductal papillary mucinous neoplasm (IPMN), islet cell tumors); penile cancer (e.g., Paget's disease of the penis and scrotum); pinealoma; primitive neuroectodermal tumor (PNT); plasma cell neoplasia; paraneoplastic syndrome; intraepithelial neoplasm; prostate cancer (e.g., prostate adenocarcinoma); rectal cancer; rhabdomyosarcoma; salivary gland cancer; skin cancer (e.g., squamous cell carcinoma (SCC), keratoacanthoma (KA), melanoma, basal cell carcinoma (BCC)); small bowel cancer (e.g., appendix cancer); soft tissue sarcoma (e.g., malignant fibrous histiocytoma (MFH), liposarcoma, malignant peripheral nerve sheath tumor (MPNST), chondrosarcoma, fibrosarcoma, myxosarcoma); sebaceous gland carcinoma; small intestine cancer; sweat gland carcinoma, synovioma, testicular cancer (e.g., seminoma, testicular embryonal carcinoma); thyroid cancer (e.g., papillary carcinoma of the thyroid, papillary thyroid carcinoma (PTC), medullary thyroid cancer); urethral cancer; vaginal cancer; and vulvar cancer (e.g., Paget's disease of the vulva). Specifically, the cancer cells may be derived from one or more cancers selected from the group consisting of head and neck cancer, melanoma, leukemia, breast cancer, ovarian cancer, bladder cancer, prostate cancer, lung cancer, colon cancer, glioblastoma, stomach cancer, pancreatic cancer, liver cancer, cervical cancer, carcinoid, endometrial cancer, kidney cancer, testicular cancer, glioma, thyroid cancer, skin cancer, and lymphoma. More specifically, the cancer cells may be derived from one or more cancers selected from the group consisting of head and neck cancer, melanoma, leukemia, breast cancer, ovarian cancer, bladder cancer, prostate cancer, lung cancer, colon cancer, and glioblastoma.

In the present disclosure, when the antigen protein is derived from cancer cells, the antigen protein may be one or more selected from the group consisting of MAGE 1/2/3, WT1, CDK4, MUC-1, HER2, PSA, HPV16 E6/E7, and HCV.

In the present disclosure, to acquire better chemical stability, enhanced pharmacological properties (half-life, absorption, potency, efficacy, or the like), altered specificity (e.g., a broad spectrum of biological activity), and reduced or enhanced antigenicity, the antigen protein may have a protecting group bound to the N- or C-terminus thereof. The protecting group may be an acetyl group, a fluorenyl methoxy carbonyl group, a formyl group, a palmitoyl group, a myristyl group, a stearyl group, or polyethylene glycol (PEG), but may include any component capable of modifying the antigen protein, particularly increasing the stability of the antigen protein, without limitation.

The term "stability" may mean not only *in vivo* stability, which protects the antigen protein from attack by protein-cleaving enzymes *in vivo,* but also storage stability (e.g., storage stability at room temperature).

In the present disclosure, the antigen protein may also be derived from natural origin, and may be obtained by various polypeptide synthesis methods widely known in the art. For example, the antigen protein may be produced by using genetic recombination and a protein expression system or may be produced by an *in vitro* synthesis method through chemical synthesis such as protein synthesis (e.g., liquid or solid phase synthesis, fragment condensation, F-MOC or T-BOC chemistry), a cell-free synthesis method, and the like. In addition, for example, the antigen protein may be a peptide, an extract of a plant-derived tissue or cell, or a product obtained by culturing a microorganism (e.g., bacteria or fungi, and particularly, yeast).

In the present disclosure, when the antigen protein is derived from a human immunodeficiency virus, the gene encoding the antigen protein may be a gene encoding a p24 protein derived from human immunodeficiency virus-1.

Also, in the present disclosure, when the antigen protein is derived from hepatitis B virus, the gene encoding the antigen protein may be a gene encoding an s protein derived from hepatitis B virus.

In addition, in the present disclosure, when the antigen protein is derived from influenza virus, the gene encoding the antigen protein may be a gene encoding one or more proteins selected from the group consisting of a H1N1 protein, a H2N2 protein, a H3N2 protein, a H5N1 protein, a H7N7 protein, a H7N9 protein, a H9N2 protein, and a H10N7 protein, which are derived from influenza virus, specifically a gene encoding a H1N1 protein derived from influenza virus, and more specifically, may be a polynucleotide encoding a polypeptide consisting of the amino acid sequence of SEQ ID NO: 1.

Also, when the antigen protein is derived from respiratory syncytial virus, the gene encoding the antigen protein may be a gene encoding an RSV-F protein derived from respiratory syncytial virus, and specifically, may be a polynucleotide encoding a polypeptide consisting of the amino acid sequence of SEQ ID NO: 2.

Also, in the present disclosure, when the antigen protein is derived from varicella-zoster virus, the antigen protein may be a gene encoding glycoprotein E derived from varicella-zoster virus, and specifically, may be a polynucleotide encoding a polypeptide consisting of the amino acid sequence of SEQ ID NO: 3.

In addition, in the present disclosure, the gene encoding the cancer cell-derived antigen protein may be a gene encoding one or more proteins selected from the group consisting of MAGE 1/2/3, WT1, CDK4, MUC-1, HER2, PSA, HPV16 E6/E7, and HCV.

In the present disclosure, the gene encoding the antigen protein may be, for example, DNA, RNA, or a combination or variant thereof, and may be double-stranded or single-stranded. In addition, to acquire better chemical stability, enhanced pharmacological properties (half-life, absorption, potency, efficacy, and the like), altered specificity (e.g., a broad spectrum of biological activity), and reduced or enhanced antigenicity, the gene encoding the antigen protein may encode an amino acid sequence of the antigen protein having a protecting group bound to the N- or C-terminus thereof, and the protecting group may include any component capable of enhancing, in particular, stability without limitation.

In addition, in the present disclosure, the antigen protein or the gene encoding the antigen protein may be loaded into the extracellular vesicle.

A method of loading the antigen protein or the gene encoding the antigen protein into an extracellular vesicle is not particularly limited and may be appropriately selected by those of ordinary skill in the art from methods known in the relevant technical field. For example, the antigen protein may be loaded into an extracellular vesicle after the extracellular vesicle is treated with extracorporeal shockwaves.

The term "extracellular vesicle" as used herein refers to a small sphere surrounded by a membrane derived from cells, and the name of these spheres varies greatly depending on the origin of a cell from which the spheres are made or a method by which the spheres are made. In the present disclosure, the extracellular vesicle may include one or more selected from the group consisting of exosomes, ectosomes, microvesicles, bacterial outer membrane vesicles (bacterial OMVs), microorganism-derived extracellular vesicles, including yeast-derived extracellular vesicles and the like, and apoptotic bodies, and specifically, may be, but is not limited to, exosomes or bacterial OMVs.

The extracellular vesicle may be an extracellular vesicle derived from one or more selected from the group consisting of a natural organism, e.g., animal, plants, and microorganisms, or an artificially produced extracellular vesicle. Also, the cells may be cells isolated from a natural organism subject. In addition, the cells may be derived from any type of animal or plant, including humans and non-human mammals. The animal cells may be one or more selected from the group consisting of somatic cells, germ cells, immune cells, neurons, and tumor cells, specifically immune cells, and more specifically activated immune cells.

Also, in the present disclosure, when the extracellular vesicle is derived from a microorganism, the microorganism may be a bacterium, and the extracellular vesicle may be a bacterial outer membrane vesicle.

In the present disclosure, the animal cells may be one or more selected from the group consisting of Vero, MDCK, MRC-5, serum-free Hi-5, Hi-5, WI-38, PER.C6, HEK-293, and CEF.

The somatic cells are cells that constitute the body of any type of animal including mammals including humans. In the present disclosure, the somatic cells may include all types of cells other than germ cells, immune cells, neurons, and tumor cells.

The immune cells refer to cells that determine the immune response in the body, that is, cells that serve to defend against invading pathogens, foreign substances, viruses, and the like, control immunity and remove cancer cells constantly produced in the body. Specifically, the immune cells may include, but are not limited to, natural killer (NK) cells, dendritic cells, T cells, B cells, macrophages, and lymphocytes.

The neurons refer to cells that constitute the nervous system, that is, cells that may express ion channels such as sodium and potassium channels to transmit signals in an electrical manner unlike other cells and exchange various types of information by exchanging chemical signals with other adjacent neurons through a structure called a synapse. In the present disclosure, the specific type of the neurons is not limited, and may include all types of cells that secrete extracellular vesicles.

The tumor cells are used with the same meaning as cancer cells, and refer to cells whose cell cycle is not regulated unlike normal cells, resulting in rapid and irregular growth of the cells. In the present disclosure, the origin tissue of the tumor cells is not particularly limited, and the tumor cells may include all types of tumor cells that secrete extracellular vesicles.

In the present disclosure, when the extracellular vesicle is derived from animal cells, the extracellular vesicle may express one or more proteins selected from the group consisting of CD9, CD63, CD81, Alix, TSG101, Syntenin 1, and Flotillin 1.

Also, in the present disclosure, when the extracellular vesicle is derived from plant cells, the extracellular vesicle may express one or more proteins selected from the group consisting of Syntaxin (PEN1), Tetraspanin 8 (Tet8), and Heat shock protein (HSP).

In the present disclosure, when the extracellular vesicle is derived from a microorganism, the extracellular vesicle may express one or more proteins selected from the group consisting of OmpA, Flagellin, HSP70, and beta-glucan. Specifically, when the microorganism is a bacterium, the extracellular vesicle may express one or more proteins selected from the group consisting of OmpA and Flagellin, and when the microorganism is a fungus, the extracellular vesicle may express one or more proteins selected from the group consisting of HSP70 and beta-glucan.

Also, the extracellular vesicle may be derived from activated immune cells, and specifically, may be derived from THP-1 cells or KG-1 cells. In this regard, the activation may be realized by lipopolysaccharide (LPS) or α-galactosylceramide (α-GC). When THP-1 cells are activated, the THP-1 cells may express one or more proteins selected from the group consisting of IL-1b, IL-6, IL-8, TNF-α, and iNOS while changing their cell morphology similar to macrophages, and specifically, may express all of IL-1b, IL-6, IL-8, TNF-α, and iNOS.

In specific embodiments of the present disclosure, for example, the phenotypic change of immune cells activated through antigen stimulation was analyzed, the characteristics of exosomes isolated therefrom were observed, and outer membrane vesicles were isolated from attenuated bacteria, and the characteristics thereof were observed.

Specifically, in an embodiment of the present disclosure, it was confirmed that, when THP-1 cells, which are a human monocyte cell line, were treated with lipopolysaccharide (LPS), the morphology of the cells was changed and markers for M1 macrophages were expressed, compared to cells which were not treated with LPS (see Example 1).

In another embodiment of the present disclosure, it was confirmed that the activated immune cell-derived exosomes, preferably activated THP-1 cell-derived exosomes, had an average diameter of 128.0 mm, and expressed exosome marker proteins, such as CD63, CD81, and Flotillin 1 (see Example 2).

In another embodiment of the present disclosure, the culture broth of an attenuated bacterium BL21 strain was centrifuged to collect the supernatant, and the supernatant was allowed to pass through a filter, concentrated and washed to produce outer membrane vesicles (OMVs). It was confirmed that, as a result of analyzing the characteristics of the OMVs through NTA, the average diameter of the OMVs was 119.2 nm, and the concentration of the isolated OMVs was 1.5 x 10¹¹ particles/ml, and as a result of quantitative analysis of protein concentration through a BCA assay, the protein concentration was 2.2 mg/ml (see Example 3).

In another embodiment of the present disclosure, activated THP-1 cell-derived exosomes loaded with an influenza virus-derived H1N1 protein or a respiratory syncytial virus-derived RSV-F protein were produced and subcutaneously administered to mice to immunize the mice, and then whether an antibody is produced was analyzed, and as a result, it was confirmed that, compared to when an adjuvant and the H1N1 protein or the RSV-F protein were administered together, the exosomes exhibited a similar level or a much higher level of H1N1- or RSV-F-specific IgG formation in a much smaller amount (see Examples 4 and 6).

In another embodiment of the present disclosure, activated THP-1 cell-derived exosomes loaded with influenza virus-derived H1N1 mRNA, respiratory syncytial virus-derived RSV-F mRNA, or varicella-zoster virus-derived glycoprotein E mRNA, or bacterial outer membrane vesicles loaded with influenza virus-derived H1N1 mRNA, respiratory syncytial virus-derived RSV-F mRNA or varicella-zoster virus-derived glycoprotein E mRNA were produced (see Example 5).

In another embodiment of the present disclosure, the activated THP-1 cell-derived exosomes or bacterial outer membrane vesicles loaded with influenza virus-derived H1N1 mRNA, respiratory syncytial virus-derived RSV-F mRNA, or varicella-zoster virus-derived glycoprotein E mRNA, which were produced in Example 5, were subcutaneously administered to mice to immunize the mice, and then whether an antibody is produced was analyzed. As a result, it was confirmed that both the activated THP-1 cell-derived exosomes or the bacterial outer membrane vesicles exhibited a high level of H1N1-, RSV-F- or VZE-specific IgG formation, compared to a control (see Example 7-1).

In another embodiment of the present disclosure, the activated THP-1 cell-derived exosomes or bacterial outer membrane vesicles loaded with influenza virus-derived H1N1 mRNA, respiratory syncytial virus-derived RSV-F mRNA or varicella-zoster virus-derived glycoprotein E mRNA, which were produced in Example 4, were subcutaneously administered to mice to immunize the mice, and then spleen cells were isolated from the immunized mice and an H1N1- or VZE antigen-specific T cell response was analyzed by the amount of IFN-γ. As a result, it was confirmed that both the mice immunized with the activated THP-1 cell-derived exosomes and the mice immunized with the bacterial outer membrane vesicles induced a strong T cell response compared to a control (see Example 7-2).

Since it was confirmed that, when mice were immunized through administration of the extracellular vesicle according to the present disclosure, the extracellular vesicle has an excellent effect of inducing the production of an antigen-specific antibody and a T cell response, the extracellular vesicle according to the present disclosure can be effectively used in the field of development of a vaccine for the prevention or treatment of various diseases, including viral infection, microbial infection, or cancer.

In the present disclosure, the viral infection may be one or more selected from the group consisting of adenovirus infection, smallpox virus infection, polio virus infection, measles virus infection, hepatitis C virus infection, human immunodeficiency virus-1 (HIV-1) infection, hepatitis B virus (HBV) infection, influenza virus infection, respiratory syncytial virus infection, herpes simplex virus infection, human papilloma virus infection, zika virus infection, varicella-zoster virus infection, and severe fever with thrombocytopenia syndrome virus infection. Specifically, the viral infection may be one or more selected from the group consisting of human immunodeficiency virus-1 (HIV-1) infection, hepatitis B virus (HBV) infection, influenza virus infection, respiratory syncytial virus infection, and varicella-zoster virus infection. More specifically, the viral infection may be one or more selected from the group consisting of influenza virus infection, varicella-zoster virus infection, and respiratory syncytial virus infection.

In addition, in the present disclosure, the microbial infection may be infection caused by a microorganism of one or more genera selected from *Salmonella, Yersinia, Escherichia, Chlamydia, Xanthomonas, Erwinia, Pseudomonas, Ralstonia, Vibrio, Neisseria, Mycobacterium, Streptococcus, Staphylococcus, Enterococcus, Lactobacillus, Aspergillus, Blastomyces, Ajellomyces, Candida, Coccidioides, Cryptococcus, Histoplasma, Rhizopus, Mucor, Cunninghamella, Apophysomyces, Absidia, Saksenaea, Entomophthora, Conidiobolus, Basidiobolus, Sporothrix, Pneumocystis, Talaromyces, Asclepias, Fusarium, Scedosporium,* and *Mucorales.*

In addition, in the present disclosure, the cancer may be one or more selected from the group consisting of: acoustic neuroma; adenocarcinoma; adrenal gland cancer; anal cancer; angiosarcoma (e.g., lymphangiosarcoma, lymphangioendotheliosarcoma, hemangiosarcoma); appendix cancer; benign monoclonal gammopathy; biliary cancer (e.g., cholangiocarcinoma); bladder cancer; breast cancer (e.g., adenocarcinoma of the breast, papillary carcinoma of the breast, mammary cancer, medullary carcinoma of the breast); brain cancer (e.g., meningioma, glioblastoma, glioblastoma (e.g., astrocytoma, oligodendroglioma), medulloblastoma); bronchus cancer; carcinoid tumor; cervical cancer (e.g., cervical adenocarcinoma); choriocarcinoma; chordoma; craniopharyngioma; colorectal cancer (e.g., colon cancer, rectal cancer, colorectal adenocarcinoma); connective tissue cancer; epithelial carcinoma; ependymoma; endotheliosarcoma (e.g., Kaposi's sarcoma, multiple idiopathic hemorrhagic sarcoma); endometrial cancer (e.g., uterine cancer, uterine sarcoma); esophageal cancer (e.g., adenocarcinoma of the esophagus, Barrett's adenocarcinoma); Ewing's sarcoma; ocular cancer (e.g., intraocular melanoma, retinoblastoma); familiar hypereosinophilia; gall bladder cancer; gastric cancer (e.g., stomach adenocarcinoma); gastrointestinal stromal tumor (GIST); germ cell cancer; head and neck cancer (e.g., head and neck squamous cell carcinoma), oral cancer (e.g., oral squamous cell carcinoma), throat cancer (e.g., laryngeal cancer, pharyngeal cancer, nasopharyngeal cancer, oropharyngeal cancer); heavy chain disease (e.g., alpha chain disease, gamma chain, disease, mu chain disease); hemangioblastoma; hypopharynx cancer; inflammatory myofibroblastic tumors; immunocytic amyloidosis; kidney cancer (e.g., nephroblastoma, also known as Wilms' tumor, renal cell carcinoma); liver cancer (e.g., hepatocellular cancer (HCC), malignant hepatoma); lung cancer (e.g., bronchogenic carcinoma, small cell lung cancer (SCLC), non-small cell lung cancer (NSCLC), adenocarcinoma of the lung); leiomyosarcoma (LMS); mastocytosis (e.g., systemic mastocytosis); muscle cancer; myelodysplastic syndrome (MDS); mesothelioma; myeloproliferative disorder (MPD) (e.g., polycythemia vera (PV), essential thrombocytosis (ET), agnogenic myeloid metaplasia (AMM) of unknown etiology, also known as myelofibrosis (MF), chronic idiopathic myelofibrosis, chronic myelocytic leukemia (CML), chronic neutrophilic leukemia (CNL), hypereosinophilic syndrome (HES); neuroblastoma; neurofibroma (e.g., neurofibromatosis (NF) type 1 or type 2, schwannomatosis; neuroendocrine cancer (e.g., gastroenteropancreatic neuroendocrine tumor (GEP-NET), carcinoid tumor; soteosarcoma (e.g., bone cancer); ovarian cancer (e.g., cystadenocarcinoma, ovarian embryonal carcinoma, ovarian adenocarcinoma); papillary adenocarcinoma; pancreatic cancer (e.g., pancreatic andenocarcinoma, intraductal papillary mucinous neoplasm (IPMN), islet cell tumors); penile cancer (e.g., Paget's disease of the penis and scrotum); pinealoma; primitive neuroectodermal tumor (PNT); plasma cell neoplasia; paraneoplastic syndrome; intraepithelial neoplasm; prostate cancer (e.g., prostate adenocarcinoma); rectal cancer; rhabdomyosarcoma; salivary gland cancer; skin cancer (e.g., squamous cell carcinoma (SCC), keratoacanthoma (KA), melanoma, basal cell carcinoma (BCC)); small bowel cancer (e.g., appendix cancer); soft tissue sarcomas (e.g., malignant fibrous histiocytoma (MFH), liposarcoma, malignant peripheral nerve sheath tumor (MPNST), chondrosarcoma, fibrosarcoma, myxosarcoma); sebaceous gland carcinoma; small intestine cancer; sweat gland carcinoma, synovioma, testicular cancer (e.g., seminoma, testicular embryonal carcinoma); thyroid cancer (e.g., papillary carcinoma of the thyroid, papillary thyroid carcinoma (PTC), medullary thyroid cancer); urethral cancer; vaginal cancer; and vulvar cancer (e.g., Paget's disease of the vulva). Specifically, the cancer may be one or more selected from the group consisting of head and neck cancer, melanoma, leukemia, breast cancer, ovarian cancer, bladder cancer, prostate cancer, lung cancer, colon cancer, glioblastoma, stomach cancer, pancreatic cancer, liver cancer, cervical cancer, carcinoid, endometrial cancer, kidney cancer, testicular cancer, glioma, thyroid cancer, skin cancer, and lymphoma. More specifically, the cancer may be one or more selected from the group consisting of head and neck cancer, melanoma, leukemia, breast cancer, ovarian cancer, bladder cancer, prostate cancer, lung cancer, colon cancer, and glioblastoma.

Also, the present disclosure provides a vaccine composition for preventing or treating viral infection, including an extracellular vesicle which includes a virus-derived antigen protein or a gene encoding the virus-derived antigen protein.

The terms "virus-derived antigen protein," "gene encoding the protein," "extracellular vesicle," "virus," "viral infection," and the like may be as described above.

The term "prevention" as used herein refers to any action that suppresses or delays the onset of a specific disease or condition via administration of the vaccine composition according to the present disclosure.

The term "treatment" as used herein refers to any action in which the symptoms for a specific disease or condition are improved or beneficially changed via administration of the vaccine composition according to the present invention.

The term "vaccine" as used herein is intended to include all types of actions of preventing infection or reinfection by a corresponding pathogen or antigen, reducing the severity of symptoms or eliminating the symptoms, or substantially or completely eliminating a disease caused by the corresponding pathogen or antigen by inducing an immune response against the corresponding antigen in a host including humans. Therefore, the vaccine composition of the present disclosure is preferably prophylactically administered to a subject prior to infection with the corresponding pathogen or the onset of a disease, and may also be administered therapeutically after infection with the corresponding pathogen or the onset of the disease.

Also, in the present disclosure, the vaccine composition may induce antigen-specific antibody production and a T cell-mediated immune response at the same time.

In an embodiment of the present disclosure, after activated THP-1 cell-derived exosomes loaded with an influenza virus-derived H1N1 protein or a respiratory syncytial virus-derived RSV-F protein were produced and subcutaneously administered to mice to immunize the mice, whether to produce an antibody was analyzed, and as a result of the analysis, it was confirmed that, compared to when an adjuvant and the H1N1 protein or the RSV-F protein were administered together, the exosomes exhibited a similar level or a much higher level of H1N1- or RSV-F-specific IgG formation in a much smaller amount.

In another embodiment of the present disclosure, the activated THP-1 cell-derived exosomes loaded with influenza virus-derived H1N1 mRNA, respiratory syncytial virus-derived RSV-F mRNA, or varicella-zoster virus-derived glycoprotein E mRNA, or the bacterial outer membrane vesicles, which were produced in Example 5, were subcutaneously administered to mice to immunize the mice, and whether an antibody is produced was analyzed. As a result of the analysis, it was confirmed that both the activated THP-1 cell-derived exosomes or the bacterial outer membrane vesicles exhibited a high level of H1N1-, RSV-F- or VZE-specific IgG formation, compared to a control.

In another embodiment of the present disclosure, the activated THP-1 cell-derived exosomes loaded with influenza virus-derived H1N1 mRNA, respiratory syncytial virus-derived RSV-F mRNA, or varicella-zoster virus-derived glycoprotein E mRNA, or the bacterial outer membrane vesicles, which were produced in Example 5, were subcutaneously administered to mice to immunize the mice, and then splenocytes were isolated from the immunized mice and an H1N1- or VZE antigen-specific T cell response was analyzed by the amount of IFN-γ. As a result, it was confirmed that both the mice immunized with the activated THP-1 cell-derived exosomes and the mice immunized with the bacterial outer membrane vesicles induced a strong T cell response compared to a control.

In addition, in the present disclosure, the vaccine composition may be freeze-dried.

The inventors of the present disclosure confirmed the specific immune response induction and storage stability of the vaccine composition. Specifically, to evaluate the storage stability of the extracellular vesicle according to the present disclosure, freeze-dried exosomes were stored for 7 days under a refrigerated condition of 4 °C, or stored at room temperature, and mice were then subcutaneously immunized to analyze whether the antibody is produced. As a result, it was confirmed that excellent stability and an excellent antibody production effect were maintained even after the exosomes were stored under the above conditions.

The immune cell-derived exosomes activated through examples of the present disclosure show lymph node orientation after subcutaneous injection. Because the immune cell-derived exosomes are mainly absorbed by local macrophages and dendritic cells, an MHC-I/peptide complex is observed in exosomes by dendritic cells. From the results, it can be seen that immune cell-derived exosomes may more effectively produce a neutralizing antibody against a virus, and may also induce a H1N1-, VZE- or RSV-F-specific T cell response in splenocytes of the immunized mice.

Therefore, the results confirmed in examples of the present disclosure suggest that nano-sized extracellular vesicles including exosomes secreted from cells or bacterial outer membrane vesicles can become a platform capable of serving as a potent immunostimulant and an antigen carrier to design an effective vaccine against a target disease.

The vaccine composition of the present disclosure may be prepared in any suitable form of a pharmaceutically acceptable formulation. For example, the vaccine composition may be prepared in the form of a ready-to administer solution or suspension, in a concentrated stock solution suitable for dilution prior to administration, or in a reconstitutable form such as a lyophilized, freeze-dried, or frozen formulation.

The vaccine composition of the present disclosure may be formulated by further including a pharmaceutically acceptable carrier. Specifically, the carrier may be, for example, a colloidal suspension, powder, saline, a lipid, liposomes, microspheres, or nanospherical particles. These may form a complex with a vehicle or may be associated with the vehicle, and may be delivered *in vivo* by using delivery systems known in the art, such as lipids, liposomes, microparticles, gold, nanoparticles, polymers, condensation reagents, polysaccharides, polyamino acids, dendrimers, saponins, adsorption enhancing substances or fatty acids.

In addition, the pharmaceutically acceptable carrier will generally include a diluent, an excipient, a stabilizer, a preservative, and the like. Suitable diluents may include nonaqueous solvents such as propylene glycol, polyethylene glycol, vegetable oils such as olive oil and peanut oil, and the like, or aqueous solvents such as saline (preferably, 0.8% saline), water containing a buffered medium (preferably, a 0.05 M phosphate buffer), and the like. Suitable excipients may include starch, glucose, lactose, sucrose, gelatin, malt, rice, wheat flour, chalk, silica gel, sodium stearate, glycerol monostearate, talc, sodium chloride, anhydrous skim milk, glycerol, propylene, glycol, water, ethanol, and the like. Suitable stabilizers may include carbohydrates such as sorbitol, mannitol, starch, sucrose, dextran, glutamate, glucose, and the like, or proteins such as animal, vegetable or microbial proteins such as milk powder, serum albumin, casein, and the like. Suitable preservatives may include thimerosal, merthiolate, gentamicin, neomycin, nystatin, amphotericin B, tetracycline, penicillin, streptomycin, polymyxin B, and the like.

The vaccine composition of the present disclosure may further include an antigen adjuvant. The antigen adjuvant may consist of one or more substances that enhance an immune response against an antigen. For example, the antigen adjuvant may be one or more selected from the group consisting of Freund's complete adjuvant, Freund's incomplete adjuvant, a saponin, a gel-phase aluminum adjuvant, a surface active substance (e.g., lysolecithin, plurone glycol, a polyanion, a peptide, an oil, a hydrocarbon emulsion, or the like), vegetable oil (e.g., cottonseed oil, peanut oil, corn oil, or the like), vitamin E acetate, squalene, GLA-SE, muramyl dipeptide, lipopolysaccharide (LPS), cuyl A, aluminum salts (Alum), Al(OH)₃, and α-galactosylceramide (α-GC). Specifically, the antigen adjuvant may be Al(OH)₃.

The vaccine composition of the present disclosure may be prepared using a method commonly used in the art to which the present disclosure pertains. The vaccine composition may be prepared as an oral or parenteral formulation, and the parenteral formulation may be preferably administered via any one route of administration selected from the group consisting of transdermal, intramuscular, intraperitoneal, intravenous, subcutaneous, nasal, or eidural route.

The term "administration" refers to an action of introducing a predetermined substance into a subject using a suitable method, and the term "subject" refers to any living organism including a human, a mouse, a rat, livestock, and the like, capable of having a viral infection. As a specific example, the subject may be a mammal including a human.

The vaccine composition of the present disclosure may be administered in a pharmaceutically effective amount. The term "pharmaceutically effective amount" as used herein refers to an amount sufficient to exhibit a vaccine effect, that is, an amount that does not cause side effects nor produce a serious or excessive immune response, and a level of the effective dose may vary depending on various factors including a disorder to be treated, the severity of the disorder, the activity of a vaccine substance, a route of administration, the rate of protein clearance, the duration of treatment, the substance used in combination with or concurrently with the vaccine composition, the age, weight, gender, diet, general health condition of the subject, and other factors known in the medical field.

Also, the present disclosure provides a vaccine composition for preventing or treating a microbial infection, including an extracellular vesicle which includes a microorganism-derived antigen protein or a gene encoding the microorganism-derived antigen protein.

The terms "microorganism-based antigen protein," "gene encoding the microorganism-based antigen protein," "extracellular vesicle," "microorganism," "microbial infection," "prevention," "treatment," "vaccine composition," and the like may be as described above.

Also, the present disclosure provides a vaccine composition for preventing or treating cancer, including an extracellular vesicle which includes a cancer cell-derived antigen protein or a gene encoding the protein.

The terms "cancer cell-derived antigen protein," "gene encoding the cancer cell-derived antigen protein," "extracellular vesicle," "cancer cell," "cancer," "prevention," "treatment," "vaccine composition," and the like may be as described above.

Also, the present disclosure provides a health functional food for preventing or ameliorating a viral infection, including an extracellular vesicle which includes a virus-derived antigen protein or a gene encoding the virus-derived antigen protein.

The terms "virus-derived antigen protein," "gene encoding the virus-derived antigen protein," "extracellular vesicle," "virus," "viral infection," "prevention," and the like may be as described above.

The term "amelioration" may refer to any action that reduces at least the severity of parameters, for example, symptoms associated with the condition to be treated. In this regard, the health functional food may be used simultaneously with or separately from a therapeutic drug before or after the onset of the corresponding disease to prevent or ameliorate a viral infection.

The health functional food of the present disclosure may further include an antigen adjuvant. For example, the antigen adjuvant may be one or more selected from the group consisting of Freund's complete adjuvant, Freund's incomplete adjuvant, a saponin, a gel-phase aluminum adjuvant, a surface active substance (e.g., lysolecithin, plurone glycol, a polyanion, a peptide, an oil, a hydrocarbon emulsion, or the like), vegetable oil (e.g., cottonseed oil, peanut oil, corn oil, or the like), vitamin E acetate, squalene, GLA-SE, muramyl dipeptide, lipopolysaccharide (LPS), cuyl A, aluminum salts (Alum), Al(OH)₃, and α-galactosylceramide (α-GC). Specifically, the antigen adjuvant may be Al(OH)₃.

In the health functional food, the active ingredient may be added directly to food, or used together with other foods or food ingredients, and may be appropriately used according to conventional methods. The mixing amount of the active ingredient may be appropriately determined depending on the purpose of use (for prevention or amelioration). In general, the health functional food may be specifically added in an amount of about 15 wt% or less, more specifically about 10 wt% or less with respect to raw materials during the preparation of foods or beverages. However, when the health functional food is taken for an extended period for the purpose of health and hygiene or for the purpose of health control, the amount may be below the above range.

The health functional food may further include one or more of a carrier, a diluent, an excipient, and an additive, and may be formulated as one selected from the group consisting of a tablet, a pill, powder, a granule, powder, a capsule, and a liquid formulation. Foods to which the compound according to an embodiment may be added may include various foods, powders, granules, tablets, capsules, syrups, beverages, gum, teas, vitamin complexes, health functional foods, and the like.

Specific examples of the carrier, the excipient, the diluent, and the additive may be at least one selected from the group consisting of lactose, dextrose, sucrose, sorbitol, mannitol, erythritol, starch, gum acacia, calcium phosphate, alginate, gelatin, calcium phosphate, calcium silicate, microcrystalline cellulose, polyvinylpyrrolidone, cellulose, polyvinylpyrrolidone, methylcellulose, water, sugar syrup, methylcellulose, methyl hydroxybenzoate, propyl hydroxybenzoate, talc, magnesium stearate, and mineral oil.

The health functional food may contain other ingredients as essential ingredients without any particular limitation, in addition to the active ingredient. For example, as in conventional beverages, the health functional food may contain various flavoring agents or natural carbohydrates as additional ingredients. Examples of the above-described natural carbohydrates may include: monosaccharides such as glucose, fructose, and the like; disaccharides such as maltose, sucrose, and the like; and polysaccharides, for example, conventional sugars such as dextrin, cyclodextrin, and the like, and sugar alcohols such as xylitol, sorbitol, erythritol, and the like. Other than the flavoring agents described above, natural flavoring agents (thaumatin, a stevia extract (e.g., rebaudioside A, glycyrrhizin, and the like)), and synthetic flavoring agents (saccharin, aspartame, and the like) may be advantageously used. The proportion of the natural carbohydrate may be appropriately determined through selection by those of ordinary skill in the art.

In addition, the health functional food according to an aspect may contain various nutrients, vitamins, minerals (electrolytes), flavoring agents such as synthetic and natural flavoring agents, coloring agents and thickening agents (cheese, chocolate, and the like), pectic acid and salts thereof, alginic acid and salts thereof, organic acids, protective colloidal thickeners, pH adjusters, stabilizers, preservatives, glycerin, alcohols, carbonation agents used in carbonated beverages, and the like. These components may be used alone or in combination, and the proportion of these additives may also be appropriately selected by those of ordinary skill in the art.

Also, the present disclosure provides a health functional food for preventing or ameliorating a microbial infection, including an extracellular vesicle which includes a microorganism-derived antigen protein or a gene encoding the microorganism-derived antigen protein.

The terms "microorganism-derived antigen protein," "gene encoding the microorganism-derived antigen protein," "extracellular vesicle," "microorganism," "microbial infection," "prevention," "amelioration," "health functional food," and the like may be as described above.

Also, the present disclosure provides a health functional food for preventing or ameliorating a viral infection, including an extracellular vesicle which includes a cancer cell-derived antigen protein or a gene encoding the cancer cell-derived antigen protein.

The terms "cancer cell-based antigen protein," "gene encoding the cancer cell-based antigen protein," "extracellular vesicle," "cancer cell," "cancer," "prevention," "amelioration," "health functional food," and the like may be as described above.

Also, the present disclosure provides a method of preventing or treating a viral infection, including a step of administering an extracellular vesicle which includes a virus-derived antigen protein or a gene encoding the virus-derived antigen protein to a subject in need thereof.

The terms "virus-derived antigen protein," "gene encoding the virus-derived antigen protein," "extracellular vesicle," "subject," "administration," "viral infection," "prevention," "treatment," and the like may be as described above.

Also, the present disclosure provides a method of preventing or treating a microbial infection, including a step of administering an extracellular vesicle which includes a microorganism-derived antigen protein or a gene encoding the microorganism-derived antigen protein to a subject in need thereof.

The terms "microorganism-derived antigen protein," "gene encoding the microorganism-derived antigen protein," "extracellular vesicle," "subject," "administration," "microbial infection," "prevention," "treatment," and the like may be as described above.

Also, the present disclosure provides a method of preventing or treating cancer, including a step of administering an extracellular vesicle which includes a cancer cell-derived antigen protein or a gene encoding the cancer cell-derived antigen protein to a subject in need thereof.

The terms "cancer cell-derived antigen protein," "gene encoding the cancer cell-derived antigen protein," "extracellular vesicle," "subject," "administration," "cancer," "prevention," "treatment," and the like may be as described above.

Also, the present disclosure provides use of an extracellular vesicle for the preparation of a drug for preventing or treating a viral infection, the extracellular vesicle including a virus-derived antigen protein or a gene encoding the virus-derived antigen protein.

The terms "virus-derived antigen protein," "gene encoding the virus-derived antigen protein," "extracellular vesicle," "viral infection," "prevention," "treatment," and the like may be as described above.

Also, the present disclosure provides use of an extracellular vesicle for the preparation of a drug for preventing or treating a microbial infection, the extracellular vesicle including a microorganism-derived antigen protein or a gene encoding the microorganism-derived antigen protein.

The terms "microorganism-derived antigen protein," "gene encoding the microorganism-derived antigen protein," "extracellular vesicle," "microbial infection," "prevention," "treatment," and the like may be as described above.

Also, the present disclosure provides use of an extracellular vesicle for the preparation of a drug for preventing or treating cancer, the extracellular vesicle including a cancer cell-derived antigen protein or a gene encoding the cancer cell-derived antigen protein.

The terms "cancer cell-derived antigen protein," "gene encoding the cancer cell-derived antigen protein," "extracellular vesicle," "cancer," "prevention," "treatment," and the like may be as described above.

### Mode for Invention

Hereinafter, exemplary examples will be provided to aid in understanding of the present disclosure. However, the following examples are only provided for easier understanding of the present disclosure and are not intended to limit the scope of the present disclosure.

### Examples

### Example 1. Observation of phenotype of activated immune cells

To develop a vaccine using extracellular vesicles, the inventors of the present disclosure first selected immune cells and stimulated the immune cells with an antigen to prepare a vaccine using exosomes isolated from activated immune cells. To this end, THP-1 cells, which are a human monocyte cell line, as an example of the immune cells were activated, and the phenotype of the activated cells was observed.

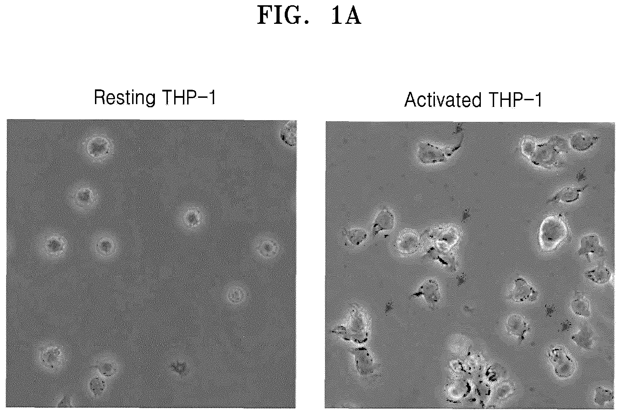

Specifically, THP-1 cells (8x10⁵ cells/mL) were treated with 100 ng/mL of lipopolysaccharide (LPS) to activate the cells, and then the cell morphologies of the treated THP-1 cells and resting THP-1 cells not treated with LPS were observed by using a microscope. As a result, as illustrated in FIG. 1A, it was confirmed that the cell morphology of the activated THP-1 cells was changed in a manner similar to macrophages, compared to the resting THP-1 cells.

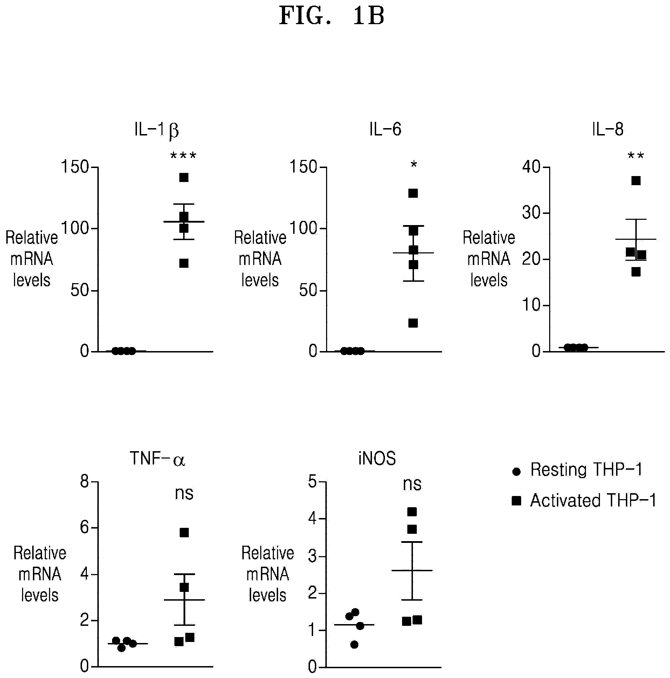

Furthermore, based on the above results, the mRNA levels of pro-inflammatory cytokine markers IL-1b, IL-6, IL-8, TNF-α, and iNOS secreted by M1 macrophages were measured to confirm whether the THP-1 cells activated by the LPS treatment differentiated into M1 macrophages, as reported in the related art. As a result, as illustrated in FIG. 1B, it was confirmed that the markers were hardly expressed in the case of the resting THP-1 cells, whereas the expression of the markers increased, and particularly, the mRNA levels of IL-1b, IL-6, and IL-8 significantly increased in the case of the activated THP-1 cells.

From the above results, it can be seen that the THP-1 cells activated through the LPS treatment exhibited the M1 macrophage phenotype.

### Example 2. Characterization of activated immune cell-derived exosomes

To confirm the characteristics of the activated THP-1 cell-derived exosomes, the inventors of the present disclosure isolated exosomes from the cells through the following process. Specifically, to remove cells and cell debris, a cell culture medium was centrifuged at 1,200 rpm for 5 minutes to collect the supernatant. Thereafter, the collected supernatant was allowed to pass through a 0.2 µm filter to remove microparticles larger than exosomes, and then the exosomes were concentrated and washed by using a Tangential flow filtration (TFF) system.

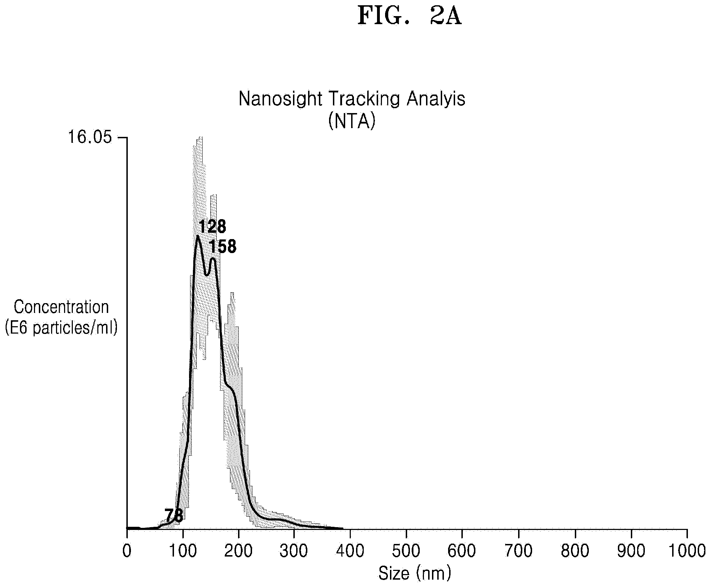

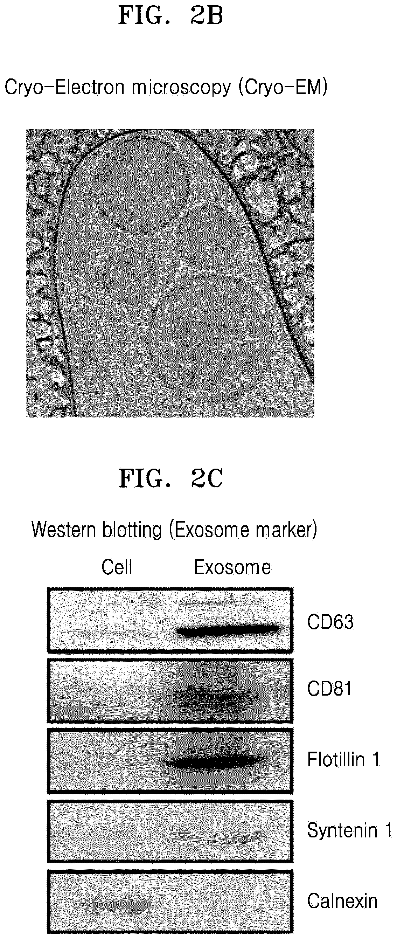

Next, the characteristics of the isolated exosomes were analyzed through Nanosight tracking analysis (NTA) and cryo-electron microscopy. As a result, as illustrated in FIGS. 2A and 2B, it was confirmed that the isolated exosomes had an average diameter of 128.0 nm and a circular shape.

In addition, the concentration of the isolated exosomes was 2.6 x 10¹¹ particles/ml, and it was confirmed through a BCA assay that, as a result of quantitative analysis, the concentration of the exosome protein was 3.5 mg/ml. Furthermore, to examine whether the isolated exosomes express exosome markers, Western blotting was performed using a cell lysate and the isolated exosomes to measure the expression levels of exosome markers CD63, CD81, and Flotillin 1. As a result, as shown in FIG. 2C, it was confirmed that only CD63 was expressed at a weak level in the cell lysate, but all of the marker proteins were expressed at high levels in the isolated exosomes.

From the above results, it can be clearly seen that the exosomes isolated from the activated THP-1 cells were exosomes having the characteristics described above.

### Example 3. Characterization of attenuated bacteri-derived outer membrane vesicles

To confirm the characteristics of the attenuated bacterium (BL21 strain)-derived outer membrane vesicles (OMVs), the inventors of the present disclosure isolated OMVs from the bacterium through the following process. Specifically, to remove the bacterium and bacterium debris, a culture broth was centrifuged at 3,000 rpm for 15 minutes to collect the supernatant. Thereafter, the collected supernatant was allowed to pass through a 0.2 um filter to remove large microparticles, and then the OMVs were concentrated and washed by using a Tangential flow filtration (TFF) system.

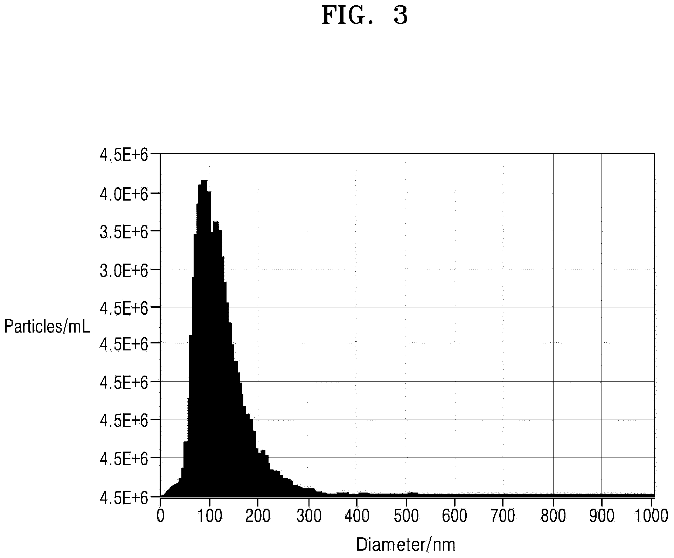

Next, the characteristics of the isolated OMVs were analyzed by observation through Nanosight tracking analysis (NTA). As a result, as illustrated in FIG. 3, it was confirmed that the average diameter of the isolated OMVs was 119.2 nm, and the concentration of the isolated OMVs was 1.5 x 10¹¹ particles/ml, and as a result of quantitative analysis through a BCA assay, the protein concentration was 2.2 mg/ml.

### Example 4. Preparation of exosomes loaded with recombinant virus-derived protein

The inventors of the present disclosure loaded the activated THP-1 cell-derived exosomes isolated in Example 2 with an influenza virus-derived H1N1 protein or a respiratory syncytial virus-derived RSV-F protein to verify the prophylactic effect thereof as a vaccine against a viral infection.

To this end, first, a construct of the influenza virus-derived H1N1 protein (A/Puerto Rico/8/1934; Accession # ABD77675.1) and a construct of the respiratory syncytial virus-derived RSV-F (Fusion glycoprotein; Accession # P03420.1) protein were prepared. Thereafter, to load the activated THP-1 cell-derived exosomes with the H1N1 protein or RSV-F protein expressed from the construct, 1.4 x 10¹¹ (3 mg) of exosomes and 50 µg of the H1N1 protein or the RSV-F protein were prepared and treated with extracorporeal shockwaves (ESWs). Then, the H1N1 protein- or RSV-F protein-loaded exosomes were washed and concentrated by using an ultracentrifuge (at 32,000 rpm for 16 hours).

Next, the amount of the H1N1 protein or RSV-F protein loaded into the THP-1 cell-derived exosomes was quantitatively analyzed by using ELISA. As a result, it was confirmed that a total of 41.1 µg of the H1N1 protein was loaded into the exosomes, and a total of 36.43 µg of the RSV-F protein was loaded into the exosomes. The inventors of the present disclosure attempted to evaluate the effect of the activated THP-1 cell-derived exosomes (ImmunExo H1N1) loaded with an influenza virus-derived H1N1 protein or the activated THP-1 cell-derived exosomes (ImmunExo RSV-F) loaded with a respiratory syncytial virus-derived RSV-F protein, which were prepared above, on a viral infection through the following experiments.

### Example 5. Preparation of extracellular vesicles loaded with recombinant virus-derived mRNA

The inventors of the present disclosure loaded the activated THP-1 cell-derived exosomes isolated in Example 2 or bacterial outer membrane vesicles (bacterial OMVs) isolated in Example 3 with influenza virus-derived H1N1 (Influenza A H1N1 Hawaii/70/2019 Hemagglutinin) mRNA, respiratory syncytial virus-derived RSV-F (Human respiratory syncytial virus (RSV) (A2) Fusion glycoprotein) mRNA, and varicella-zoster virus glycoprotein E (VZE) mRNA to verify the prophylactic effect thereof as a vaccine against a viral infection.

Specifically, the mRNA for each protein was produced by rnagene from a DNA template through an *in vitro* transcription (IVT) process. For the produced mRNAs, THP-1 cell-derived exosomes or bacterial OMVs (6 mg) and 100 µg of each mRNA were prepared and treated with extracorporeal shockwaves (ESWs). Then, the extracellular vesicles loaded with mRNA of each virus-derived protein were washed and concentrated by using an ultracentrifuge (at 32,000 rpm for 2 hours).

Next, the amount of each mRNA loaded into the THP-1 cell-derived exosomes or the bacterial OMVs was quantitatively analyzed by using qRT-PCR. As a result, it was confirmed that a total of 67.08 µg, 60.3 µg, and 66.3 µg of H1N1, RSV-F, and VZE were loaded into the THP-1 cell-derived exosomes, and a total of 64.36 µg, 66.5 µg, and 65 µg of H1N1, RSV-F, and VZE were loaded into the bacterial OMVs. The inventors of the present disclosure attempted to evaluate the effect of the activated THP-1 cell-derived exosomes or bacterial OMVs loaded with mRNA of each virus-derived protein on each viral infection through the following experiments.

### Example 6. Confirmation of vaccine efficacy against virus of activated immune cell-derived exosomes loaded with virus-derived protein

The inventors of the present disclosure attempted to evaluate the efficacy of the activated THP-1 cell-derived exosomes prepared in Example 4, which were loaded with an influenza virus-derived H1N1 protein or a respiratory syncytial virus-derived RSV-F protein, as a vaccine against a virus.

### (1)Analysis of serum antibody response to virus in THP-1 cell-derived exosomes loaded with influenza-derived H1N1 protein

The inventors of the present disclosure obtained serum samples after 14 days from each of the mice to which 5 µg of the activated THP-1 cell-derived exosomes (ImmunExo) loaded with an influenza virus-derived H1N1 protein, which were prepared in Example 4, were administered or to which 500 µg of Al(OH)₃ as an adjuvant and 5 µg of the H1N1 protein were administered together, and then diluted the serum samples with PBS supplemented with 1% BSA at a ratio of 1:100-1:1000, and measured absorbance to analyze the degree of antibody binding in a serum specific to the influenza virus-derived H1N1 protein.

As a result, as illustrated in FIG. 4A, it was confirmed that the group administered with exosomes together with the H1N1 protein exhibited a similar degree of antibody binding in a serum specific to the influenza virus H1N1 even in a much smaller amount, to that of the group administered with an adjuvant and the H1N1 protein in combination.

### (2) Analysis of serum antibody response to virus in THP-1 cell-derived exosomes loaded with respiratory syncytial virus-derived RSV-F protein

The inventors of the present disclosure obtained serum samples after 14 days from each of the mice to which 8 µg of the activated THP-1 cell-derived exosomes (ImmunExo) loaded with a respiratory syncytial virus-derived RSV-F protein, which were prepared in Example 4, were administered or to which 500 µg of Al(OH)₃ as an adjuvant and 8 µg of the RSV-F protein were administered together, and then diluted the serum samples with PBS supplemented with 1% BSA at a ratio of 1:100-1:1000, and measured absorbance to analyze the degree of antibody binding in a serum specific to the respiratory syncytial virus-derived RSV-F protein.

As a result, as illustrated in FIG. 4B, it was confirmed that the group administered with the exosomes together with the RSV-F protein exhibited a higher degree of antibody binding in a serum specific to the respiratory syncytial virus RSV-F even in a much smaller amount, than that of the group administered with adjuvant and the RSV-F protein in combination.

### Example 7. Confirmation of vaccine efficacy against virus of extracellular vesicles loaded with mRNA of virus-derived protein

The inventors of the present disclosure attempted to evaluate the efficacy of the activated THP-1 cell-derived exosomes or bacterial outer membrane vesicles (OMVs) loaded with influenza virus-derived H1N1 mRNA, respiratory syncytial virus-derived RSV-F mRNA or varicella-zoster virus-derived glycoprotein E mRNA, which were prepared in Example 5, as a vaccine against a virus.

### 7-1. Analysis of serum antibody response in extracellular vesicles loaded with mRNA of virus-derived protein

### (1)In case of extracellular vesicles loaded with influenza virus-derived H1N1 mRNA

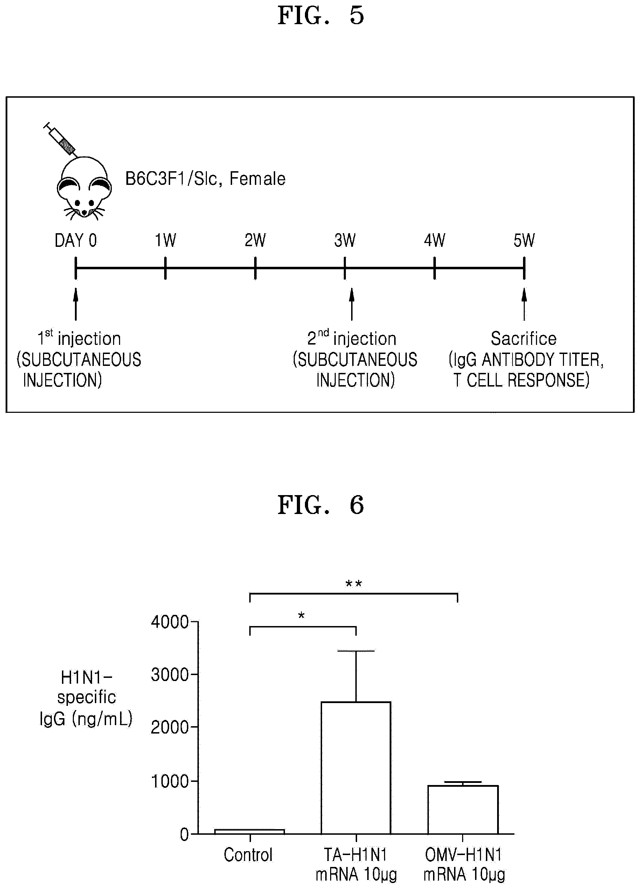

The inventors of the present disclosure administered, to B6C3F1/Slc mice (female), 10 µg of the activated THP-1 cell-derived exosomes loaded with influenza virus-derived H1N1 mRNA or the bacterial outer membrane vesicles (OMVs) loaded with influenza virus-derived H1N1 mRNA, which were prepared in Example 5, via subcutaneous injection, and after 3 weeks, administered the same extracellular vesicles in the same amount via subcutaneous injection, and then sacrificed the mice after 35 days, 5 weeks after the first administration date, thereby obtaining serum samples. Thereafter, the serum samples were diluted in PBS supplemented with 1% BSA at a ratio of 1:100-1:1000, and the degree of antibody binding in a serum specific to the influenza virus-derived H1N1 protein was analyzed through absorbance measurement (FIG. 5).

As a result, as illustrated in FIG. 6, it was confirmed that both the mice administered with the activated THP-1 cell-derived exosomes loaded with influenza virus-derived H1N1 mRNA and the mice administered with the bacterial OMVs loaded with influenza virus-derived H1N1 mRNA exhibited the degree of antibody (IgG) binding in serums specific to the influenza virus H1N1 protein, compared to a control.

### (2) In case of extracellular vesicles loaded with varicella-zoster virus-derived glycoprotein E mRNA

The inventors of the present disclosure administered, to B6C3F1/Slc mice (female), 10 µg of the activated THP-1 cell-derived exosomes loaded with varicella-zoster virus-derived glycoprotein E mRNA or the bacterial outer membrane vesicles (OMVs) loaded with varicella-zoster virus-derived glycoprotein E mRNA, which were prepared in Example 5, via subcutaneous injection, and after 3 weeks, administered the same extracellular vesicles in the same amount via subcutaneous injection, and then sacrificed the mice after 35 days, 5 weeks after the first administration date, thereby obtaining serum samples. Thereafter, the serum samples were diluted in PBS supplemented with 1% BSA at a ratio of 1:100-1:1000, and the degree of antibody binding in serums specific to the varicella-zoster virus-derived glycoprotein E was analyzed through absorbance measurement (FIG. 5).

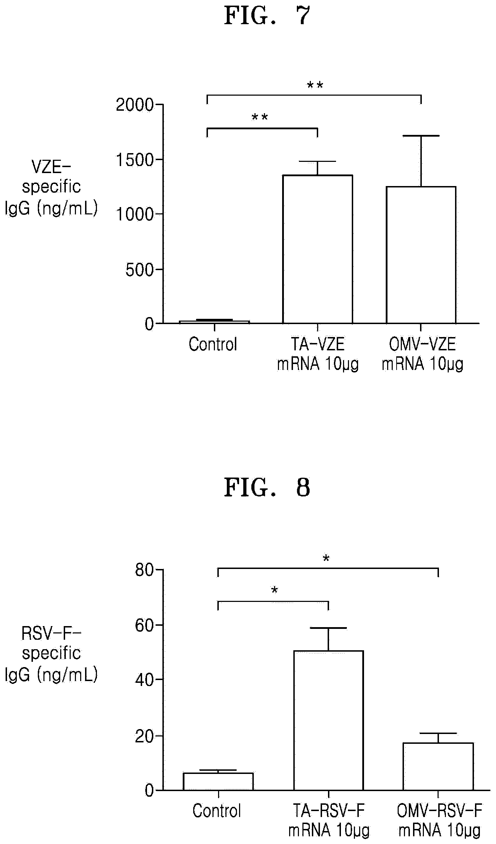

As a result, as illustrated in FIG. 7, it was confirmed that both the mice administered with the activated THP-1 cell-derived exosomes loaded with varicella-zoster virus-derived glycoprotein E mRNA and the mice administered with the bacterial OMVs loaded with varicella-zoster virus-derived glycoprotein E mRNA exhibited the degree of antibody (IgG) binding in serums specific to the varicella-zoster virus-derived glycoprotein E, compared to a control.

### (3) In case of extracellular vesicles loaded with respiratory syncytial virus-derived RSV-F mRNA

The inventors of the present disclosure administered, to B6C3F1/Slc mice (female), 10 µg of the activated THP-1 cell- derived exosomes loaded with respiratory syncytial virus-derived RSV-F mRNA or 10 µg of the bacterial outer membrane vesicles (OMVs) loaded with respiratory syncytial virus-derived RSV-F mRNA, which were prepared in Example 5, via subcutaneous injection, and after 14 days, 2 weeks after the first administration date, obtained serum samples. Thereafter, the serum samples were diluted in PBS supplemented with 1% BSA at a ratio of 1:100-1:1000, and the degree of antibody binding in serums specific to the respiratory syncytial virus-derived RSV-F protein was analyzed through absorbance measurement.

As a result, as illustrated in FIG. 8, it was confirmed that both the mice administered with the activated THP-1 cell-derived exosomes loaded with respiratory syncytial virus-derived RSV-F mRNA and the mice administered with the bacterial OMVs loaded with respiratory syncytial virus-derived RSV-F mRNA exhibited the degree of antibody (IgG) binding in serums specific to the respiratory syncytial virus-derived RSV-F protein, compared to a control.

### 7-2. Analysis of specific T cell responses to extracellular vesicles loaded with mRNA of virus-derived protein

### (1)In case of extracellular vesicles loaded with influenza virus-derived H1N1 mRNA

The inventors of the present disclosure administered, to B6C3F1/Slc mice (female), 10 µg of the activated THP-1 cell-derived exosomes loaded with influenza virus-derived H1N1 mRNA or the bacterial outer membrane vesicles (OMVs) loaded with influenza virus-derived H1N1 mRNA, which were prepared in Example 5, via subcutaneous injection, and after 3 weeks, administered the same extracellular vesicles in the same amount via subcutaneous injection, and then sacrificed the mice after 35 days, 5 weeks after the first administration date, to isolate splenocytes. Thereafter, the splenocytes (1 x 10⁷/ml) were treated with 2 µg/mL of an influenza virus-derived H1N1 protein for 48 hours, and then ELISA was performed using a culture medium of the splenocytes to measure the level of IFN-γ protein secreted from Th1 cells.

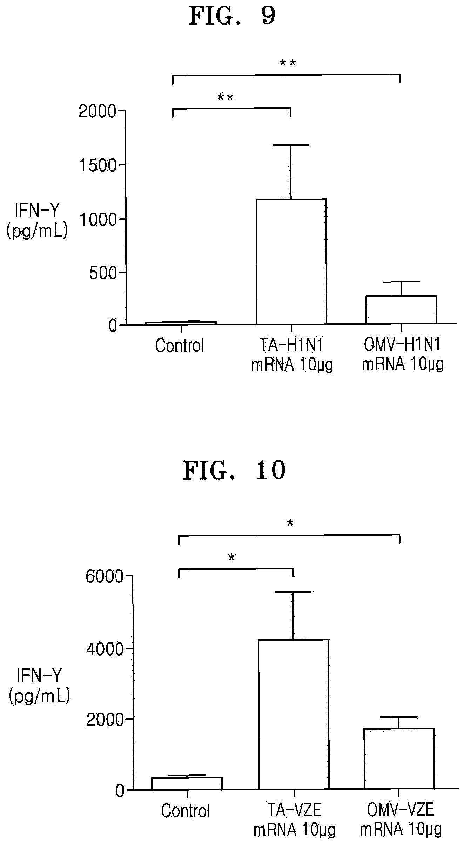

As a result, as can be seen in FIG. 9, it was confirmed that no IFN-γ protein was detected in a culture medium of a control, whereas IFN-γ was present in culture media of the splenocytes of the mice administered with the activated THP-1 cell-derived exosomes and bacterial OMVs loaded with influenza virus-derived H1N1 mRNA.

The above results suggest that a Th1 cell-mediated immune response was not induced in the control, whereas a strong Th1 cell-mediated immune response was induced by administration of the extracellular vesicles according to the present disclosure.

### (2) In case of extracellular vesicles loaded with varicella-zoster virus-derived glycoprotein E mRNA

The inventors of the present disclosure administered, to B6C3F1/Slc mice (Female), 10 µg of the activated THP-1 cell-derived exosomes loaded with varicella-zoster virus-derived glycoprotein E mRNA and 10 µg of bacterial outer membrane vesicles (OMVs) loaded with varicella-zoster virus-derived glycoprotein E mRNA, which were prepared in Example 5 via subcutaneous injection, and after 3 weeks, administered the same extracellular vesicles in the same amount via subcutaneous injection, and then sacrificed the mice after 35 days, 5 weeks after the first administration date, to isolate splenocytes. Thereafter, the splenocytes (1 x 10⁷/ml) were treated with 2 µg/mL of varicella-zoster virus-derived glycoprotein E for 48 hours, and then ELISA was performed using culture media of the splenocytes to measure the level of IFN-γ protein secreted from Th1 cells.

As a result, as illustrated in FIG. 10, it was confirmed that no IFN-γ protein was detected in a culture medium of a control, whereas IFN-γ was present in the culture media of the splenocytes of the mice administered with the activated THP-1 cell-derived exosomes and bacterial OMVs loaded with varicella-zoster virus-derived glycoprotein E mRNA.

The above results suggest that a Th1 cell-mediated immune response was not induced in the control, whereas a strong Th1 cell-mediated immune response was induced by administration of the extracellular vesicles according to the present disclosure.

The above description of the present disclosure is provided only for illustrative purposes, and it will be understood by those of ordinary skill in the art to which the present disclosure pertains that the disclosure may be embodied in various modified forms without departing from the technical spirit or essential characteristics thereof. Thus, the embodiments described herein should be considered in an illustrative sense only and not for the purpose of limitation.

## Claims

1. An extracellular vesicle comprising an antigen protein or a gene encoding the antigen protein.

2. The extracellular vesicle of claim 1, wherein the antigen protein is derived from one or more selected from the group consisting of a virus, a microorganism, and cancer cells.

3. The extracellular vesicle of claim 2, wherein the virus is one or more selected from the group consisting of adenovirus, smallpox virus, poliovirus, measles virus, hepatitis C virus, human immunodeficiency virus-1 (HIV-1), hepatitis B virus (HBV), influenza virus, respiratory syncytial virus, herpes simplex virus, human papilloma virus, zika virus, varicella-zoster virus, and severe fever with thrombocytopenia syndrome virus.

4. The extracellular vesicle of claim 1, wherein the antigen protein is one or more selected from the group consisting of a human immunodeficiency virus-1-derived p24 protein, a hepatitis B virus-derived s protein, an influenza virus-derived H1N1 protein, a respiratory syncytial virus-derived RSV-F protein, and varicella-zoster virus-derived glycoprotein E.

5. The extracellular vesicle of claim 2, wherein the microorganism is a microorganism of one or more genera selected from the group consisting of *Salmonella, Yersinia, Escherichia, Chlamydia, Xanthomonas, Erwinia, Pseudomonas, Ralstonia, Vibrio, Neisseria, Mycobacterium, Streptococcus, Staphylococcus, Enterococcus, Lactobacillus, Aspergillus, Blastomyces, Ajellomyces, Candida, Coccidioides, Cryptococcus, Histoplasma, Rhizopus, Mucor, Cunninghamella, Apophysomyces, Absidia, Saksenaea, Entomophthora, Conidiobolus, Basidiobolus, Sporothrix, Pneumocystis, Talaromyces, Asclepias, Fusarium, Scedosporium,* and *Mucorales.*

6. The extracellular vesicle of claim 2, wherein the cancer cells are derived from one or more cancers selected from the group consisting of head and neck cancer, melanoma, leukemia, breast cancer, ovarian cancer, bladder cancer, prostate cancer, lung cancer, colon cancer, and glioblastoma.

7. The extracellular vesicle of claim 1, wherein the antigen protein is one or more selected from the group consisting of MAGE 1/2/3, WT1, CDK4, MUC-1, HER2, PSA, HPV16 E6/E7, and HCV.

8. The extracellular vesicle of claim 1, wherein the antigen protein or the gene encoding the antigen protein is loaded into the extracellular vesicle.

9. The extracellular vesicle of claim 1, wherein the extracellular vesicle is derived from one or more selected from the group consisting of animal cells, plant cells, or a microorganism.

10. The extracellular vesicle of claim 9, wherein the animal cells are one or more selected from the group consisting of somatic cells, germ cells, immune cells, neurons, and tumor cells.

11. The extracellular vesicle of claim 9, wherein, when the extracellular vesicle is derived from the animal cells, the extracellular vesicle expresses one or more proteins selected from the group consisting of CD9, CD63, CD81, Alix, TSG101, Syntenin 1, and Flotillin 1.

12. The extracellular vesicle of claim 9, wherein, when the extracellular vesicle is derived from the plant cells, the extracellular vesicle expresses one or more proteins selected from the group consisting of Syntaxin (PEN1), Tetraspanin 8 (Tet8), and Heat shock protein (HSP).

13. The extracellular vesicle of claim 9, wherein, when the extracellular vesicle is derived from the microorganism, the extracellular vesicle expresses one or more proteins selected from the group consisting of OmpA, Flagellin, HSP70, and beta-glucan.

14. A vaccine composition for preventing or treating a viral infection, comprising an extracellular vesicle which comprises a virus-derived antigen protein or a gene encoding the virus-derived antigen protein.

15. A vaccine composition for preventing or treating a microbial infection, comprising an extracellular vesicle which comprises a microorganism-derived antigen protein or a gene encoding the microorganism-derived antigen protein.

16. A vaccine composition for preventing or treating cancer, comprising an extracellular vesicle which comprises a cancer cell-derived antigen protein or a gene encoding the cancer cell-derived antigen protein.

17. The vaccine composition of any one of claims 14 to 16, wherein the vaccine composition simultaneously induces the production of an antigen-specific antibody and a T cell-mediated immune response.

18. A health functional food for preventing or ameliorating a viral infection, comprising an extracellular vesicle which comprises a virus-derived antigen protein or a gene encoding the virus-derived antigen protein.

19. A health functional food for preventing or ameliorating a microbial infection, comprising an extracellular vesicle which comprises a microorganism-derived antigen protein or a gene encoding the microorganism-derived antigen protein.

20. A health functional food for preventing or ameliorating cancer, comprising an extracellular vesicle which comprises a cancer cell-derived antigen protein or a gene encoding the cancer cell-derived antigen protein.
